# EUROPEAN PATENT SPECIFICATION

(11) **EP 2 808 046 B1**
(45) Date of publication and mention of the grant of the patent: **08.11.2017**
(21) Application number: 14182680.0
(22) Date of filing: 17.09.2008
(51) Int. Cl.: A61M 5/31, A61M 5/315, A61M 5/20

(54) **A RELEASABLE CONNECTING MECHANISM**
LÖSBARER VERBINDUNGSMECHANISMUS
MÉCANISME D'ACCOUPLEMENT AMOVIBLE

(30) Priority: 21.09.2007 AU 2007905184
(43) Date of publication of application: 03.12.2014
(62) Divisional of application: 08800008.8
(73) Proprietor: Imaxeon Pty Ltd, Rydalmere, NSW 2116 (AU)
(72) Inventor: Newing, Timothy, John, Thornleigh, New South Wales 2120 (AU); Khajeh-Taheri, Shahab, Cecil Hills, New South Wales 2171 (AU)
(74) Representative: Eisenführ Speiser

(56) References cited:
- EP-A1- 1 166 807
- EP-A2- 0 308 380
- US-A- 4 677 980
- US-A- 4 911 695
- US-A- 5 007 904
- US-A1- 2007 219 508

## Description

### FIELD

The invention relates to a releasable connecting mechanism that can be used in a syringe.

### BACKGROUND

In this specification where a document, act or item of knowledge is referred to or discussed, this reference or discussion is not an admission that the document, act or item of knowledge or any combination thereof was at the priority date, publicly available, known to the public, part of common general knowledge; or known to be relevant to an attempt to solve any problem with which this specification is concerned.

In contrast injectors, replaceable syringes are used to deliver a liquid contrast media into the body of a patient. The contrast media travels in the patient's blood vessels to enable the location of the blood vessels, and any blockage in the vessels, to be detected using an X-ray, computed tomography (CT), or magnetic resonance imaging (MRI) scanner.

A syringe in this application consists of a moving element (the plunger), which propels the contrast media, and a container (the syringe barrel), which forms the body of the syringe assembly. Contrast media is typically in liquid form. The contrast media in a syringe may leak past the seal formed between the plunger and the syringe barrel. If the contrast media is left to dry, it forms a hard solid residue. This can be a problem for automated injector systems, particularly those that use replaceable syringes. In such systems, the plunger in the syringe releasably connects to a piston driven by the system, which pushes the plunger along the syringe to inject the contrast media into a patient. Any leak of contrast media past the plunger (such as during use or due to malfunction of the seal of the plunger) may come into contact with the part connecting the plunger to the piston. If the contrast media is allowed to dry, the plunger may become stuck to the piston, which makes maintenance difficult and expensive. If contrast media leaks into the connecting part of the plunger before it connects to the piston, the solid residue makes it difficult for the piston and plunger to engage or function properly, especially if the connecting part involves a mechanism with moving parts, such as retractable locking pins.

It is therefore desired to address one or more of the above, or to at least provide a useful alternative, preferably with a minimum of moving parts.

EP 0 308 380 A2 discloses a self-destroying hypodermic syringe of the disposable kind comprising a cylinder which carries a cannula at one end. In the cylinder a plunger with a plunger rod is provided, and in connection with said plunger or plunger rod a breaking means is provided to brake after the first time the syringe is used.

US 4,677,980 A discloses an angiographic injector using an injector portion which includes a rotating turret for housing two angiographic syringes in readiness for injection. The injector further employs a driving mechanism for connecting with and controlling the movement of a syringe plunger once one and of the syringes is in place for injection.

US 4,911,695 A discloses a plunger having a generally converging distal portion, and proximal face on which is mounted a coupling structure. The coupling structure is transversely engageable by, and transversely disengageable from, a driving mechanism of a power-driven angiographic syringe, and once engaged, cannot be disengaged by rotation of the driving mechanism relative to the plunger in the absence of transverse translational movement of the driving mechanism and plunger relative to one another.

US 5,007,904 A discloses a similar plunger having a generally converging distal portion, and a rear face on which is provided a coupling structure. The coupling structure is transversely engageable by, and transversely disengageable from, a driving mechanism of a power-driven angiographic syringe, and, one engaged, cannot be disengaged by rotation of the driving mechanism relative to one another.

US 2007/0219508 A1 discloses a plunger cover for use with a syringe includes a sealing portion adapted to form a seal with the syringe and a central portion adapted to contact fluid within the syringe.

EP 1 166 807 A1 discloses a medical syringe including a cylindrical syringe body and a plunger fitted in the syringe body for injecting a liquid agent filled in the syringe body from the syringe body into the vascular system of the human body by advancing the plunger relative to the syringe body. The plunger comprising a plunger body and an elastic cover fitted over the plunger body from the front.

### SUMMARY

According to the present invention, there is provided a syringe comprising:
a syringe barrel defining a main chamber; and
a plunger having a plunger body slidably disposed within the main chamber of the syringe barrel,
wherein a recess is formed on an inner wall of the plunger body, the recess configured for receiving a head portion of a piston, the recess comprising one or more radially inwardly projecting flanges on the inner wall, the plunger body having a top concave member defining a first portion of the recess and a first portion of the one or more radially inwardly projecting flanges, and a bottom ring member defining a second portion of the recess and a second portion of the one ore more radially inwardly projecting flanges, the one or more radially inwardly projecting flanges each defining an edge terminating in an apex point to define a saw-tooth shaped guide path to guide rotational movement of the head portion of the piston into the recess, and wherein the top-concave member and the bottom ring member form an interlocking engagement with each other.

Also foreseen is a releasable connecting mechanism for a syringe, said mechanism including:
a piston having a head portion with one or more radially projecting pins, said head portion being rotatable with said pins about an axis along said piston;
a plunger body having a recess formed within said body for receiving said head portion, said recess including one or more inwardly projecting flanges for guiding said pins to rotate said head portion relative to said body;
wherein, when said head portion is inserted into said recess, said flanges rotate said head portion to a locking position where said body resists disengagement from said piston, and when said head portion is further inserted into said recess, said head portion is released from said locking position and rotates to a release position allowing said piston to disengage from said body.

Also foreseen is a releasable connecting mechanism for a syringe, said mechanism including:
a piston having a head portion with one or more outwardly projecting flanges, said head portion being rotatable with said flanges about an axis along said piston;
a plunger body having a recess formed within said body for receiving said head portion, said recess including one or more inwardly projecting pins for guiding the rotation of said head portion relative to said body;
wherein, when said head portion is inserted into said recess, said pins rotate said head portion to a locking position where said body resists disengagement from said piston, and when said head portion is further inserted into said recess, said head portion is released from said locking position and rotates to a release position allowing said piston to disengage from said body.

### BRIEF DESCRIPTION OF THE DRAWINGS

Representative embodiments of the present invention are herein described, by way of example only, with reference to the accompanying drawings, wherein:
Figures 1 to 3 are block diagrams of one embodiment of the connecting mechanism;
Figures 4 to 6 are cross-sectional diagrams of one embodiment of the connecting mechanism at different stages of operation;
Figures 7 to 10 are diagrams of a top concave member of a plunger;
Figures 11 to 18 are diagrams of a bottom ring member of a plunger;
Figures 19 to 22 are diagrams of a rotatable head portion of a piston;
Figures 23 to 34 are diagrams of another embodiment of the connecting mechanism at different stages of operation;
Figures 35 to 39 are diagrams of a rotatable head portion;
Figures 40 and 41 are bottom views of a rotatable head portion;
Figures 42 to 49 are different views of a representative embodiment of the head portion; and
Figures 50 to 58 are views of a representative embodiment of the plunger as assembled.

### DETAILED DESCRIPTION OF THE REPRESENTATIVE EMBODIMENTS

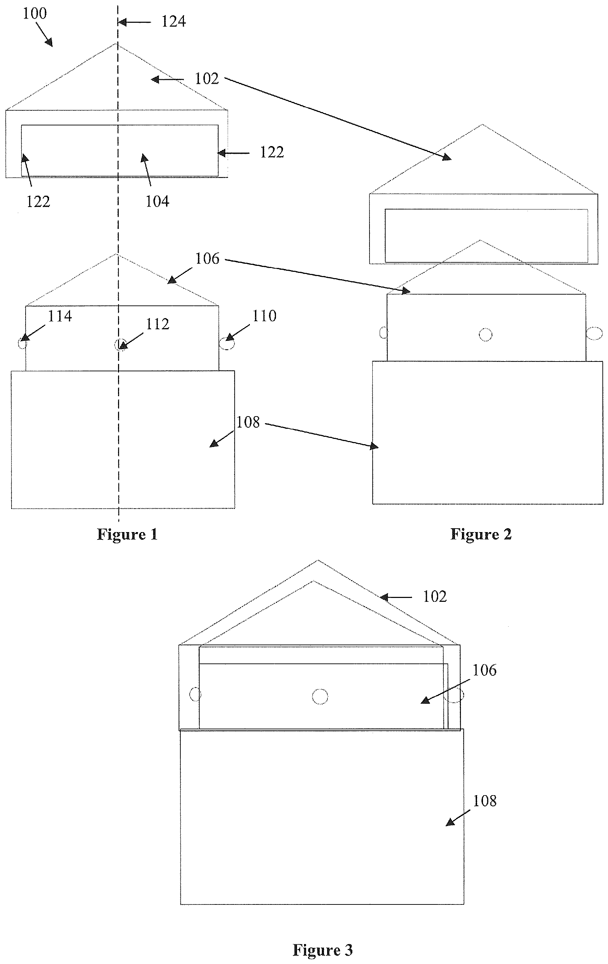

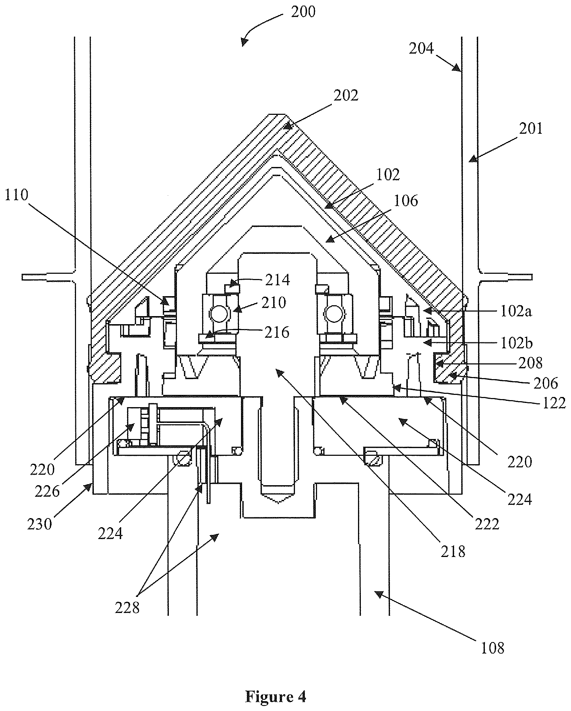

Figure 1 shows the components 100 operating inside the main chamber 200 of a syringe barrel 201 (see Figure 4), which includes a plunger body 102 and a piston 108 with a rotatable head portion 106. A resilient cap 202 (see Figure 4), which is preferably made of an elastic material such as rubber, is fitted over the plunger body 102 to form a seal with the inner surface 204 of the main chamber 200 of the syringe barrel 201. The main chamber 200 of the syringe barrel 201 stores a liquid, such as contrast media. During use, the head portion 106 of the piston 108 connects with the plunger body 102, so that the plunger body 102 can be pushed along the main chamber 200 by the piston 108 to expel any substance (e.g. contrast media) stored in the chamber 200. The connecting mechanism refers to the mechanical interface between two components (e.g. the plunger body 102 and the head portion 106 of the piston 108), which enables these components to be releasably connected to each other.

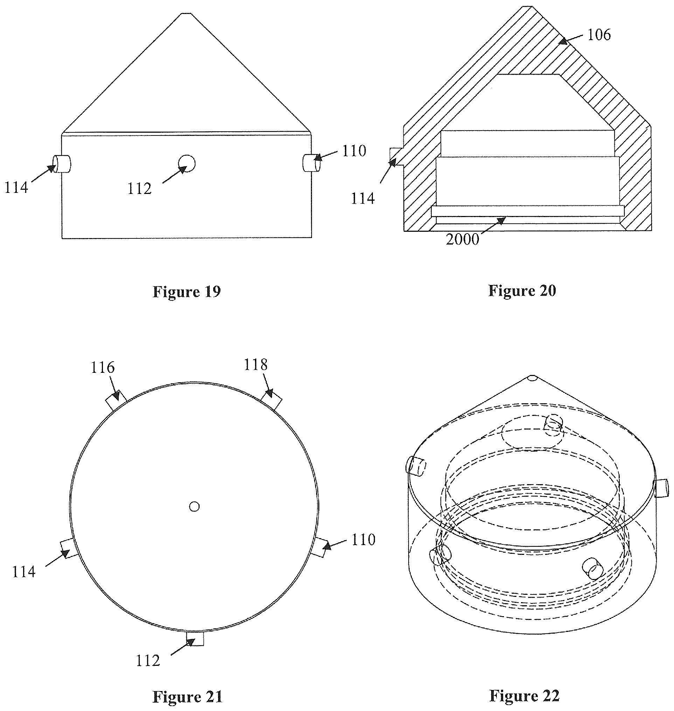

As shown in Figure 1, the piston 108 has a head portion 106 with one or more radially projecting pins 110, 112, 114, 116 and 118 (which, preferably, are radially and evenly spaced from each other, as shown in Figure 21). The head portion 106 is rotatable together with the pins 110, 112, 114, 116 and 118 about an axis 124 along the length of piston 108.

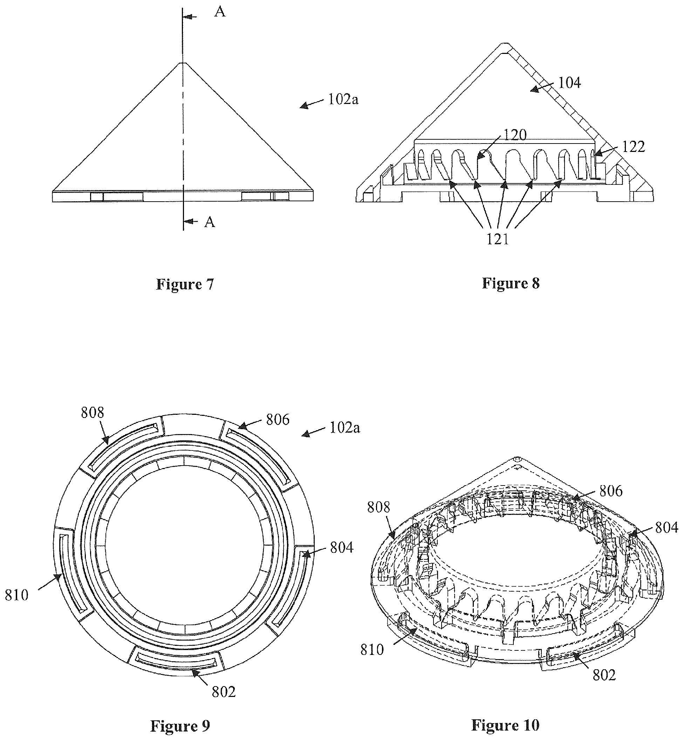

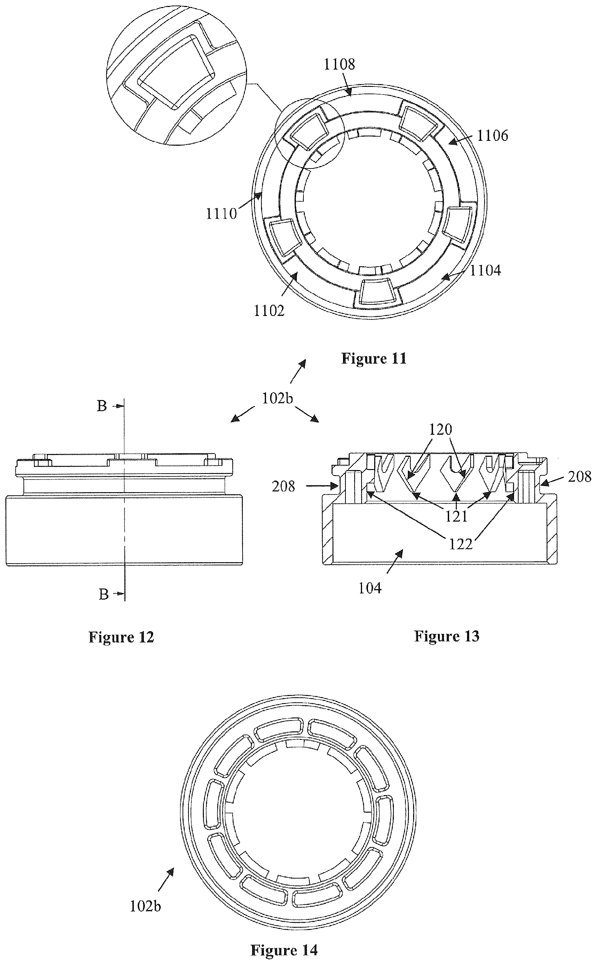

As shown in Figures 8 and 13, the plunger body 102 has a recess 104 formed within the body 102 for receiving the head portion 106. The recess 104 includes one or more inwardly projecting flanges 120 on the inner wall 122, where the edges of the flanges 120 define a path for guiding the movement of the pins 110, 112, 114, 116 and 118 to rotate the head portion 106 relative to (and inside of) the body 102.

In a representative embodiment, the connecting mechanism includes an odd number of pins 110, 112, 114, 116 and 118. In a representative embodiment, the connecting mechanism has 3 pins in total. In another representative embodiment, the connecting mechanism has 5 pins in total. The pins are preferably located on the head portion 106, or in another representative embodiment, are included as part of the plunger body 102.

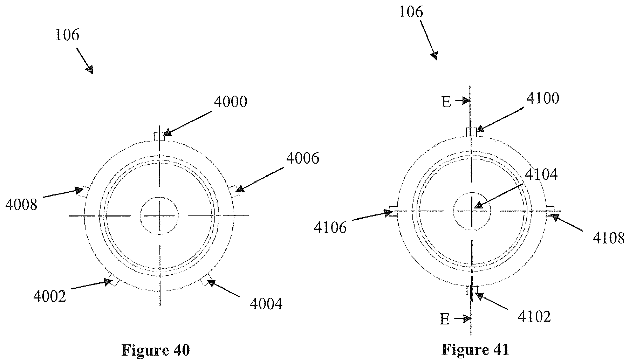

Some of the advantages from having an odd number of pins 110, 112, 114, 116 and 118 in the connecting mechanism (e.g. on the head portion) can be better understood from the examples shown in Figures 40 and 41. Figures 40 and 41 respectively show the top views of different head portions 106 with 5 and 4 radially spaced pins 4000, 4002, 4004, 4006, 4008, and 4100, 4102, 4106, 4108. If contrast media has been deposited onto one or more of the pins 4000, 4002, 4004, 4006 and 4008, and is allowed to dry or harden, the effective diameter of the contrast encrusted pins 4000, 4002, 4004, 4006 and 4008 would increase and become more likely to interfere with the cross section of the path (defined by the flanges 120) during engagement of the plunger body 102 and the head portion 106.

As shown in Figure 41, a head portion 106 with a 4-pin configuration has two natural axes of rotation, since each of the pins 4100, 4102, 4106 and 4108 has a corresponding opposing pin, which can be referred to as a pin pair. Such natural axes will exist in connecting mechanisms that have an even number of evenly spaced pins. In Figure 41, the head portion 106 normally moves in an upward direction (i.e. moving out of the page towards the reader) and rotates anti-clockwise to engage with the plunger body 102 (not shown in Figure 41). However, if a single pin of a pin pair becomes contrast encrusted (e.g. 4100), the contrast encrusted pin would initially cause some resistance to the engagement of the encrusted pin (and the head portion 106) with the plunger body 102. This may cause rotational moment to occur about the axis E-E of the perpendicular pin pair 4100 and 4102 through a central pivot point 4104 of the whole rotating piston interface bearing (e.g. pin 4100 is pushed in a direction heading into the page, which results in pin 4102 being pushed in an opposite direction heading out of the page). The other pins 4102, 4106 and 4108 that are not contrast encrusted are properly received into the respective paths (defined by the flanges 120) of the plunger body 102. These pins 4102, 4106 and 4108 exert torque that helps rotate the head portion 106 relative to the plunger body 102. When the head portion 106 is rotated in this way, the contrast encrusted pin 4100 is forced to rotate with the other pins 4102, 4106 and 4108, which forces the encrusted pin 4100 into its corresponding path in the plunger body 102. The edges of the flanges 120 that define the path help push away the contrast around the encrusted pin 4100. It can be appreciated that by having a greater number of pins 4110, 4102, 4106 and 4108, there are a greater number of places from which torque can be exerted to rotate the head portion 106.

Figure 40 relates to a head portion 106 with a 5-pin configuration that operates in the same way as the head portion 106 shown in Figure 41. The arrangement of pins 4000, 4002, 4004, 4006 and 4008 have no natural axis of rotation of the type as described with reference to in Figure 41. The result is that should a single pin 4000 become encrusted with contrast media, the opposite pin "pair" 4002 or 4004 will tend to distribute and dissipate the rotational moment of that single pin 4000. Each pin of the 5-pin version of the head portion 106 has at least one opposite pin "pair" in a triangle formation. In the example shown in Figure 40, each pin 4000, 4002, 4004, 4006 and 4008 has two other opposing pins that guide the rotation of the pin, which assists the pin to be received into the path (defined by the flanges 120) in the plunger body 102 in circumstances such as where the pin is contrast encrusted.

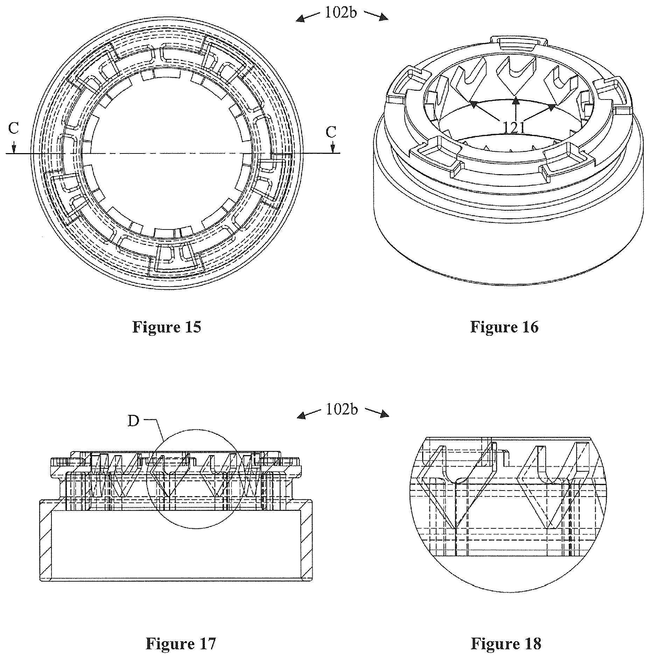

The interface of the connecting mechanism between the head portion 106 and the plunger 102 typically requires that all pins 110, 112, 114, 116 and 118 enter the path defined by the flanges 120 together (i.e. at the same time) to commence the engagement of the plunger body 102 to the head portion 106. As the head portion 106 is received into the plunger 102, all of the pins 110, 112, 114, 116 and 118 may come into contact with the lead-in path apex points 121 (see Figures 13 and 16) simultaneously, thus resulting in a pushing action on the plunger 102 rather than a rotation of the head portion 106. This problem may occur regardless of the number of pins 110, 112, 114, 116 and 118 on the head portion. Typically, however, the apex 121 of the flanges 120 on the plunger 102 are quite small, and the diameter of the pins 110, 112, 114, 116 and 118 are considerably larger than the apex 121 dimensions. Accordingly, the likelihood of this problem occurring can be reduced by ensuring that there are very tight tolerances (e.g. of the order of 0.05 mm) in the positioning of the apex points 121 to minimise the exact matching of the pin 110, 112, 114, 116 and 118 centrelines and lead-in path apex points 121.

In a representative embodiment, the entire head portion 106 is received by the recess 104, so that the mechanical interlocking arrangement between the flanges 120 and the pins 110, 112, 114, 116 and 118 operate wholly inside of the plunger body 102. This enclosed interlocking arrangement reduces the exposure of the flanges 120 and pins 110, 112, 114, 116 and 118 to substances stored inside of the syringe barrel 201 (e.g. contrast media) that may leak past the resilient cap 202. For instance, the enclosed configuration minimises the ability for contrast media to come into contact with the flanges 120 and pins 110, 112, 114, 116 and 118, which in turn, reduces the quantity of dried (or solid) contrast media that may jam or interfere with the mechanical interlocking arrangement between the flanges 120 and pins 110, 112, 114, 116 and 118 of the head portion 106 and plunger body 102. The enclosed interlocking arrangement is further enhanced by the piston's support member 224, which has a load bearing surface 222 that sits flush against a shoulder 220 of the plunger body 102 to substantially prevent the flow of a substance in the syringe (e.g. contrast media) into the space where the pins 110, 112, 114, 116 and 118 connects with the flanges 120.

After the piston 108 is released from the plunger body 102, the plunger body 102 preferably remains in the chamber 200 of the syringe barrel 201 so that the plunger body 102 can be disposed together with the syringe barrel 201. The piston 108 may be part of an injector machine that is not replaced after use with a syringe 201. During use, the substance in the chamber 200 of the syringe barrel 201 may leak past the cap 202. For example, any contrast media that leaks past the cap 202 as the syringe is being removed may come into contact with the pins 110, 112, 114, 116 and 118 (or flanges 120) on the rotating head portion 106 of the piston 108 and leave behind a solid residue on these parts (if allowed to dry). This residue makes is difficult for the head portion 106 to engage or connect to the plunger body 102 of another syringe barrel 201. The small size of the pins 110, 112, 114, 116 and 118 (or flanges 120) on the head portion 106 also make it difficult to clean these parts (e.g. by hand).

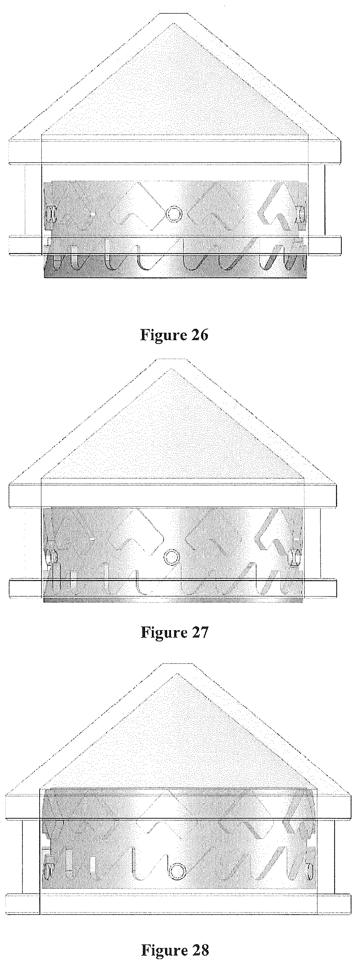

The plunger body 102 is made from a rigid material (such as polycarbonate or similar blended materials), and the recess 104 is preferably shaped to provide a close fit with the outer facing surface of the head portion 106. In the embodiments shown in Figures 1-3 and 23-34, the head portion 106 is smaller in diameter than the plunger body 102, so that the head portion 106 fits into the recess 104 of the plunger body 102 from behind. This configuration allows the plunger body 102 to push away from the head portion 106 any dried contrast media (or any other residue) on the head portion 106 when it is being received into the recess 104. In a representative embodiment, the plunger body 102 is shaped to enable the removal (or cleaning) of solid residue attached onto the head portion 106 as the two parts move towards each other for coupling together.

Figures 2 to 3 show the same component in Figure 1 in different releasable coupling configurations. In Figure 2, the plunger body 102 and piston 108 is shown moving towards each other for engagement. In Figure 3, the plunger body 102 engages the head portion 106 and connects to the piston 108.

Figures 7 to 18 are drawings of various views of the components of the plunger body 102 according to a representative embodiment. The plunger body 102 is made up of a top concave member 102a (as shown in Figures 7 to 10) that fits on top of a bottom ring member 102b (as shown in Figures 11 to 18).

Figure 7 is a side view of the top member 102a. Figure 8 is a cross-sectional view of the top member 102a along section A-A in Figure 7. Figure 9 is a bottom view of the top member 102a shown in Figure 7. Figure 10 is a perspective view of the top member 102a shown in Figure 7, where the dotted lines represent the edges inside of the top member 102a that are hidden from direct view. As shown in Figure 8, the top member 102a is shaped to define a portion of the recess 104, and also defines a portion of the inner wall 122 and flanges 120.

Figure 11 is a top view of the bottom member 102b shown in Figure 12. Figure 12 is a side view of the bottom member 102b. Figure 13 is a cross-sectional view of the bottom member 102b along section B-B in Figure 12. Figure 14 is a bottom view of the bottom member 102b shown in Figure 12. Figure 15 is another top view of the bottom member 102b shown in Figure 12, where the dotted lines represent edges of the bottom member 102b that are hidden from direct view. Figure 16 is a perspective view of the bottom member 102b shown in Figure 12. Figure 17 is another cross-sectional view of the bottom member 102b along section C-C in Figure 15, where the dotted lines represent the edges of the bottom member 102b that are not in direct view. Figure 18 is a more detailed view of Detail D in Figure 17. As shown in Figure 13, the bottom member 102b is shaped to define a portion of the recess 104, and also defines a portion of the inner wall 122 and flanges 120.

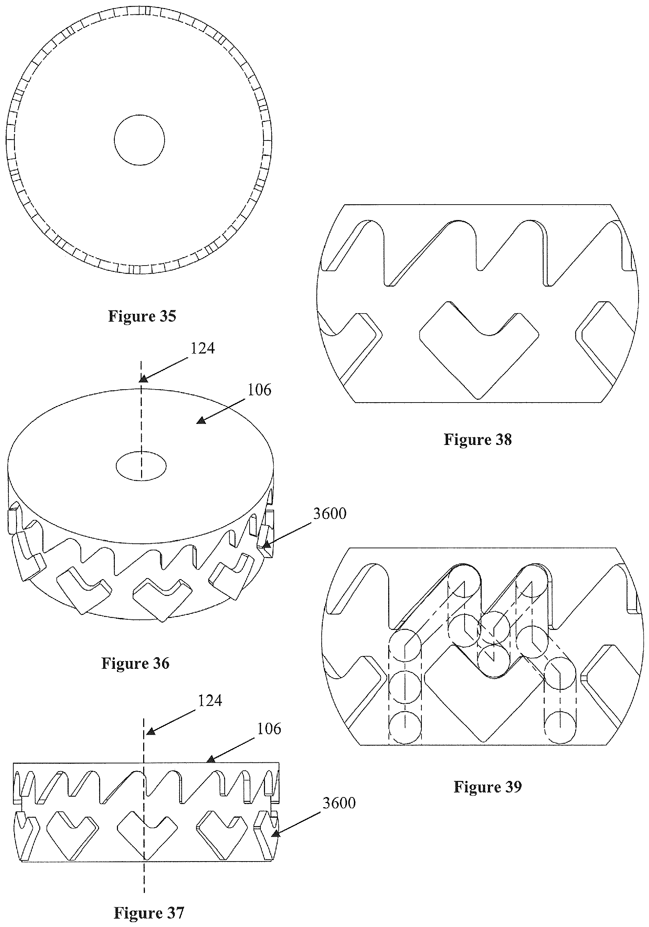

As shown in Figures 9 and 10, the lower portion of the top member 102a has one or more locating members 802, 804, 806, 808 and 810 that are radially and evenly spaced from each other along a lower perimeter of the top member 102a. The locating members 802, 804, 806, 808 and 810 are received in reciprocally shaped locating recesses 1102, 1104, 1106, 1108 and 1110, so that the top member 102a is able to form an interlocking engagement with the bottom member 102b to minimise rotation of the top and bottom members 102a and 102b relative to each other. When the top and bottom members 102a and 102b are coupled to each other, the flanges 120 on both of these members 102a and 102b combine to define a path shaped (see Figure 38) for guiding the movement of the pins 110, 112, 114, 116 and 118 (relative to the plunger body 102) along a saw tooth path in one direction (e.g. as shown in Figure 39). In another representative embodiment, the locating members 802, 804, 806, 808 and 810 are formed on the bottom member 102b, and the recesses 1102, 1104, 1106, 1108 and 1110 are formed on the top member 102a.

Figures 19 to 22 are diagrams of a rotatable head portion 106 of the piston 108. The head portion 106 is shaped to enable it to be received (and preferably wholly received) inside of the recess 104 of the plunger body 102. As shown in Figure 20, the head portion 106 has a cavity 2000 for engaging an end portion of the piston 108.

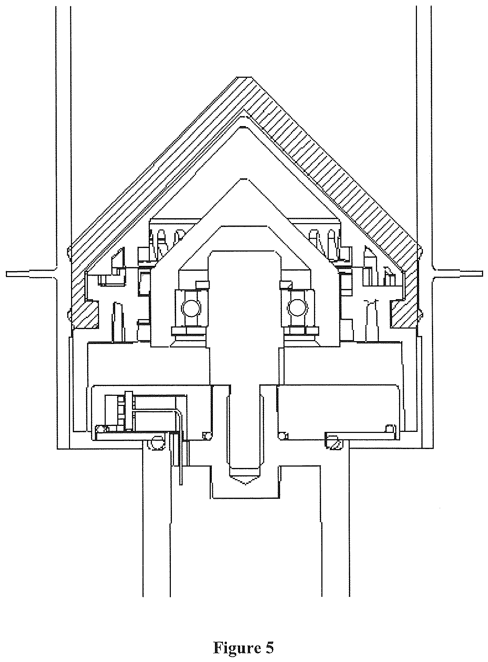

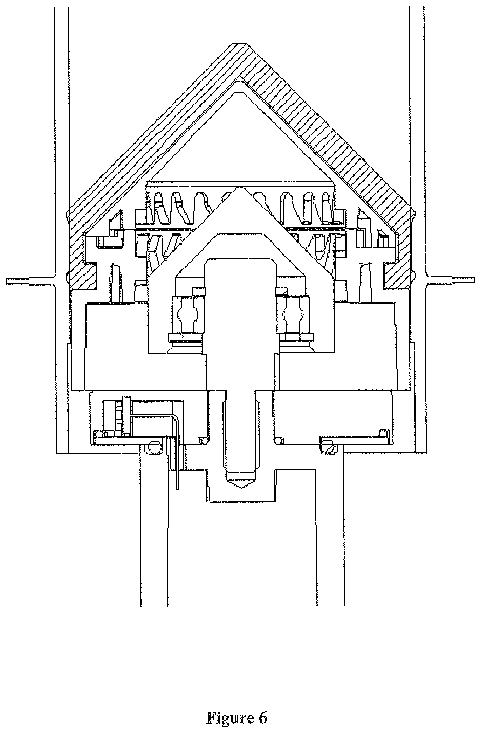

Figures 4 to 6 are cross-sectional diagrams showing a representative embodiment of the connecting mechanism at different stages of operation. Figure 4 shows the head portion 106 received wholly inside of the plunger body 102, and where the plunger body 102 and head portion 106 are in the fully engaged position (e.g. during injection). Figure 5 shows the position of the pins 110, 112, 114, 116 and 118 engaging the flanges 120 for filling. Figure 6 shows the head portion 106 fully disengaged from the plunger body 102.

As shown in Figure 4, a resilient cap 202 is fitted over the outer portion of the top and bottom members 102a and 102b. The rim 206 of the cap 202 is shaped to fit securely into a groove 208 (or recess) formed around the outer circumference of the plunger body 102 (and preferably, on the bottom ring member 102b). The top and bottom members 102a and 102b are held together by the cap 202 that clamps onto the groove 208. On a representative embodiment, the top and bottom members 102a and 102b are also glued or welded together.

As shown in Figure 4, the head portion 106 rotates about a support pin 218. Preferably, the support pin 218 is threaded at one end, and screws into (and extends from) an end portion of the piston 108. Inside the cavity 2000 of the head portion 106, the head portion 106 is coupled to a bearing 210, and the bearing 210 is rotatably coupled to the support pin 218. This enables the head portion 106 to freely rotate about the support pin 218. The bearing 210 is held in place by clip members 214 and 216 (e.g. circ-clips) located above and below the bearing 210. The bearing 210 forms a seal that minimises liquid (e.g. contrast media) from entering into the space between the head portion 106 and the support pin 218. This reduces the risk that contrast media may be allowed to dry in the space between the head portion 106 and the support pin 218, which would give rise to mechanical jamming between these parts 106 and 218.

When the head portion 106 is inserted into the recess 104, the flanges 120 rotate the head portion 106 to a locking position where the plunger body 102 resists disengagement from the piston 108. When the head portion 106 is further inserted into the recess 104 (from the locking position), the head portion 106 is released from the locking position and is allowed to rotate to a release position that allows the piston 108 to disengage from the plunger body 102.

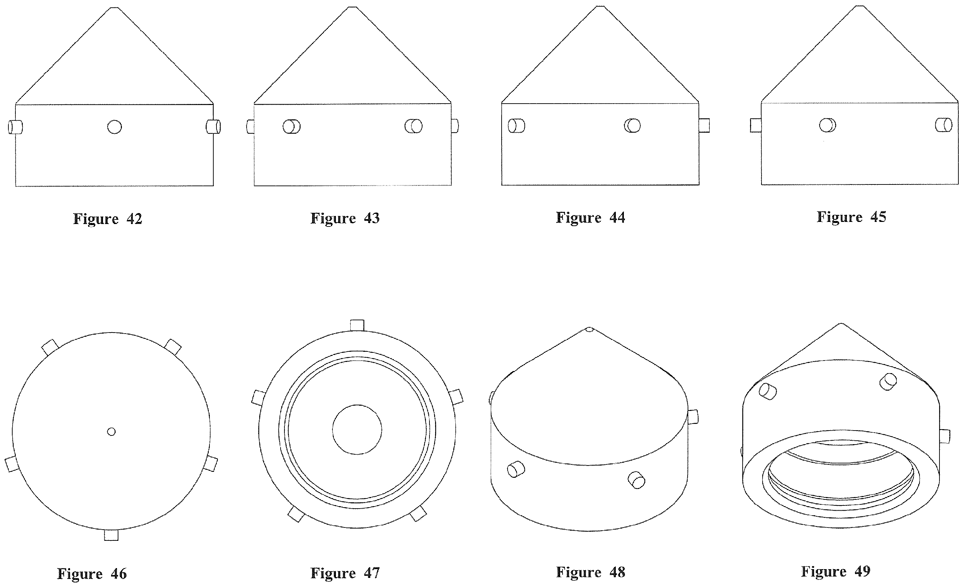

Figures 42 to 49 are different views of a representative embodiment of the rotating head portion 106 of the piston 108. In particular, Figures 42 to 49 respectively show the front view, back view, left side view, right side view, top view, bottom view, upper isometric view and lower isometric view of a representative embodiment of the head portion 106.

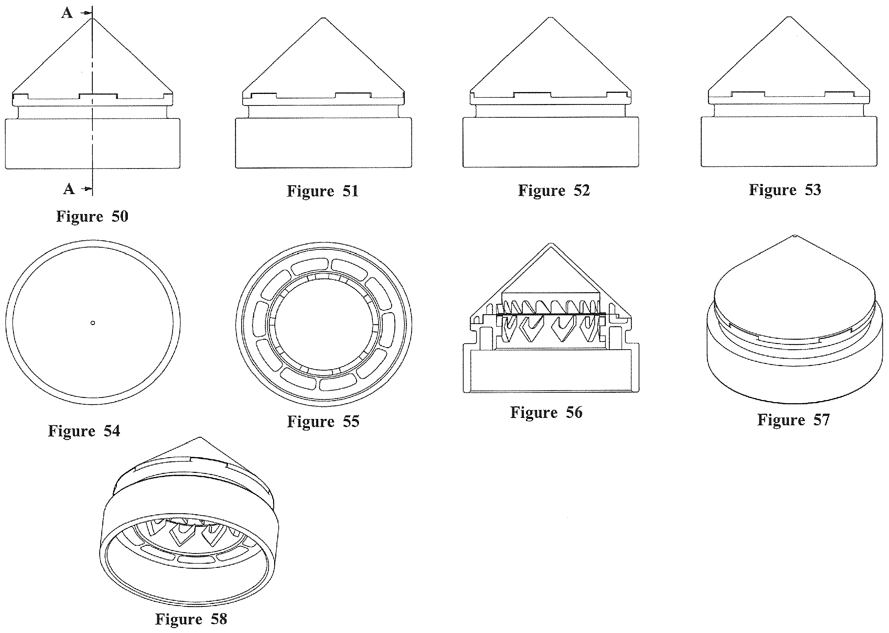

Figures 50 to 58 are views of a representative embodiment of the plunger 102 in its assembled form (i.e. when the top and bottom members 102a and 102b are held together or bonded together). In particular, Figures 50 to 58 respectively show the front view, left side view, right side view, back view, top view, bottom view, sectional view (along section A-A of Figure 42), upper isometric view and lower isometric view of a representative embodiment of the plunger 102. Figure 56 shows the representative configuration of the flanges for the embodiment shown in Figures 1 to 6.

At the locking position, the flanges 120 are shaped to receive the pins 110, 112, 114, 116 and 118 of the head portion 106 so as to prevent the head portion 106 from separating from the plunger 102 when the piston 108 moves in a rearward direction away from the plunger 102.

As shown in Figure 4, the recess 104 includes a shoulder 220, and the piston 108 includes a load bearing surface 222 for engaging the shoulder 220. The load bearing surface 222 is preferably shaped to sit flush against the shoulder 220, when the head portion 106 is fully received in the recess 104. The shoulder 220 and load bearing surface 222 provides a larger surface area on which the piston can exert force to push the plunger body 102 along the main chamber 200 of the syringe. The load bearing surface 222 is part of a support member 224. The support member 224 may be located behind the head portion 106. Preferably, the support member 224 positions the load bearing surface 222 at an angle that is substantially normal to the axis of rotation 124.

The support member 224 may include a sensor 226 for detecting the engagement and release of the head portion 106 from the plunger body 102. The sensor 226 may be a light sensor, or alternatively, the sensor 226 may be a combined infrared transceiver and receiver unit. The piston 108 includes hollow portions 228 that allow electrical and signal wiring to run to and from the sensor 226 to a control computer (not shown). The control computer receives signals from the sensor 226 representing whether the plunger body 102 is engaged or released (or separated) from the head portion 106 and plunger 102.

In a representative embodiment, the sensor 226 operates in conjunction with the outer wall 230 (or skirt) of the plunger body 102. Referring to the example shown in Figure 6, the sensor 226 is able to detect when the head portion 106 is separated from the plunger body 102, for example, by the absence of detecting any reflected infrared signal generated by the radiating element of the sensor 226. Accordingly, the sensor does not send a signal to the control computer, thus indicating that the plunger body 102 and head portion 106 are presently separated from each other. When the head portion 106 of the piston 108 is received in the plunger body 102, the outer wall 230 reflects at least some of the infrared signal generated by the sensor 226 back towards the detecting element of the sensor 226. As a result of this, the sensor 226 generates a signal to the control computer, thus indicating that the plunger body 102 and head portion 106 are coupled to each other.

The ability to detect whether the plunger body 102 is coupled to the head portion 106 can be important in some applications. For example, when the plunger body 102 is coupled to the head portion 106, it is undesirable to operate the piston in the withdrawal direction, which may result in the syringe withdrawing body fluid from a patient connected to the syringe. Also, the sensor 116 enables the control computer to control the lateral movement and strength exerted by the piston 108 and head portion 106, such as for engagement or disengagement with the plunger body 102 (with light forward or backward motion) or for exerting greater force to push the plunger body 102 along the syringe to inject material into a patient.

The outer wall 230 of the plunger body 102 is positioned proximate to an inner wall 204 of the syringe. The outer wall 230 has is sufficiently long in length so as to minimise skewing (e.g. lateral rotation) of the plunger body 102 relative to the inner wall 204 of the syringe 201 (e.g. when pushed along the syringe).

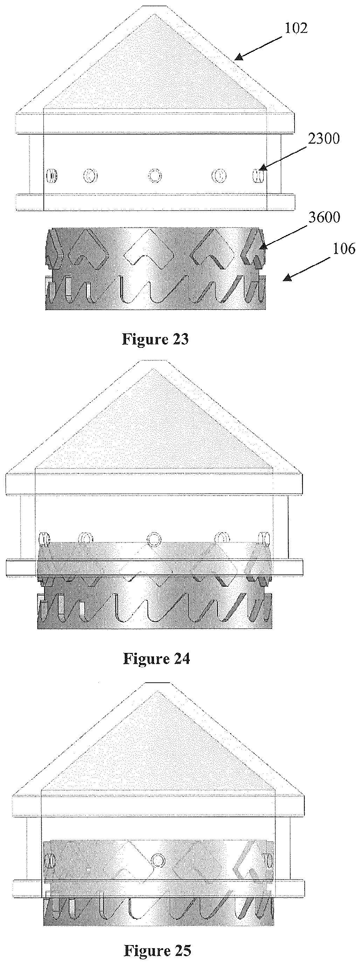

Figures 23 to 34 are diagrams of another representative embodiment of the connecting mechanism at different stages of operation. Figures 35 to 39 are diagrams of a rotatable head portion for that embodiment.

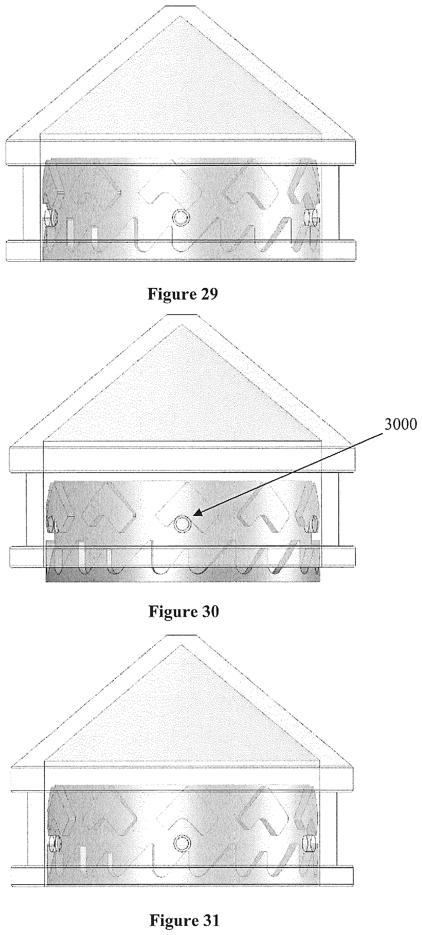

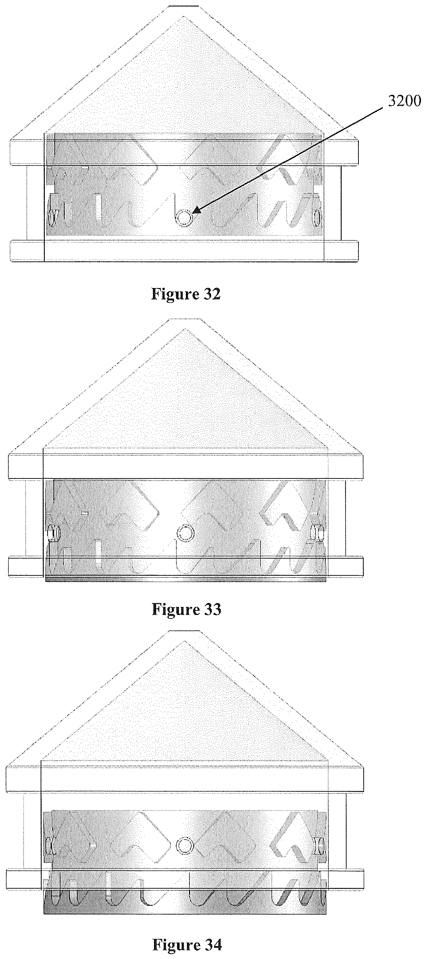

In the embodiment shown in Figures 23 to 39, the releasable connecting mechanism includes a piston 108 having a head portion 106 with one or more outwardly projecting flanges 3600. The head portion 106 is rotatable with the flanges 3600 about an axis along the piston 108. The plunger body 102 having a recess 104 formed within the plunger body 102 for receiving the head portion 106, the recess 104 including one or more inwardly projecting pins 2300 for guiding the rotation of the head portion 106 relative to the plunger body 106. Accordingly, when the head portion 106 is inserted into the recess 104, the pins 2300 rotate the head portion 106 to a locking position 3000 (see Figure 30) where the plunger body 102 resists disengagement from the piston 108. Also, when the head portion 106 is further inserted into the recess 104, the head portion 106 is released from the locking position 3000 and is allowed to rotate to a release position 3200 (see Figure 32) that allows the piston 108 to disengage from the plunger body 102.

The word 'comprising' and forms of the word 'comprising' as used in this description and in the claims does not limit the invention claimed to exclude any variants or additions.

Modifications and improvements to the invention will be readily apparent to those skilled in the art. Such modifications and improvements are intended to be within the scope of this invention.

## Claims

1. A syringe comprising:
a syringe barrel (201) defining a main chamber (200); and
a plunger having a plunger body (102) slidably disposed within the main chamber (200) of the syringe barrel (201),
**characterized in that** a recess (104) is formed on an inner wall (122) of the plunger body (102), the recess (104) configured for receiving a head portion of a piston, the recess (104) comprising one or more radially inwardly projecting flanges (120) on the inner wall (122), the plunger body (102) having a top concave member (102a) defining a first portion of the recess (104) and a first portion of the one or more radially inwardly projecting flanges (120), and a bottom ring member (102b) defining a second portion of the recess (104) and a second portion of the one ore more radially inwardly projecting flanges (120), the one or more radially inwardly projecting flanges (120) each defining an edge terminating in an apex point (121) to define a saw-tooth shaped guide path to guide rotational movement of the head portion of the piston into the recess (104), and **in that** the top concave member (102a) and the bottom ring member (102b) form an interlocking engagement with each other.

2. The syringe of claim 1, **characterized in that** the saw-tooth shaped guide path is configured for guiding one or more pins on the head portion of the piston.

3. The syringe of claim 1, **characterized in that** the one or more inwardly projecting flanges (120) have the apex point (121) configured for guiding one or more pins on the head portion of the piston into the guide path.

4. The syringe of claim 1, **characterized in that** one of the top concave member (102a) and the bottom ring member (102b) has one or more locating members (802) configured to form the interlocking engagement with a corresponding locating recess (1102) formed on the other of the top concave member (102a) and the bottom ring member (102b).

5. The syringe of claim 4, **characterized in that** the one or more locating members (802) are radially spaced from each other along a perimeter of one of the top concave member (102a) and the bottom ring member (102b).

6. The syringe of claim 1, further comprising a resilient cap (202) fitted over at least a portion of an outer portion of the plunger body (102).

7. The syringe of claim 1, further comprising a shoulder (220) formed on the recess (104) and configured for engaging a load bearing surface (220) of the piston.

## Patentansprüche

1. Spritze, umfassend:
einen Spritzenzylinder (201), der eine Hauptkammer (200) definiert; und
einen Stempel, der einen Stempelkörper (102) aufweist, welcher verschiebbar im Inneren der Hauptkammer (200) des Spritzenzylinders (201) angeordnet ist,
**dadurch gekennzeichnet, dass** eine Ausnehmung (104) an einer Innenwand (122) des Stempelkörpers (102) geformt ist, wobei die Ausnehmung (104) ausgebildet ist zum Empfangen eines Kopfteils eines Kolbens, wobei die Ausnehmung (104) ein oder mehrere radial nach innen vorspringende Flansche (120) an der Innenwand (122) umfasst, wobei der Stempelkörper (102) ein oberes konkaves Element (102a), das einen ersten Teil der Ausnehmung (104) und einen ersten Teil der ein oder mehreren radial nach innen vorspringenden Flansche (120) definiert, und ein unteres Ringelement (102b), das einen zweiten Teil der Ausnehmung (104) und einen zweiten Teil der ein oder mehreren radial nach innen überstehenden Flansche (120) definiert, aufweist, wobei jeder der ein oder mehreren radial nach innen vorspringenden Flansche (120) eine Kante definiert, die in einem Scheitelpunkt (121) endet, um einen sägezahnförmigen Führungspfad zum Führen einer Drehbewegung des Kopfteils des Kolbens in die Ausnehmung (104) zu definieren, und dadurch dass das obere konkave Element (102a) und das untere Ringelement (102b) miteinander eine ineinandergreifende Kupplung formen.

2. Spritze nach Anspruch 1, **dadurch gekennzeichnet, dass** der sägezahnförmige Führungspfad ausgebildet ist zum Führen von einem oder mehreren Stiften an dem Kopfteil des Kolbens.

3. Spritze nach Anspruch 1, **dadurch gekennzeichnet, dass** die ein oder mehreren nach innen vorspringenden Flansche (120) den Scheitelpunkt (121) aufweisen, der zum Führen von einem oder mehreren Stiften an dem Kopfteil des Kolbens in den Führungspfad hinein ausgebildet ist.

4. Spritze nach Anspruch 1, **dadurch gekennzeichnet, dass** eines von dem oberen konkaven Element (102a) und dem unteren Ringelement (102b) ein oder mehrere Fixierelemente (802) aufweist, die ausgebildet sind, die ineinandergreifende Kupplung mit einer korrespondierenden Fixierausnehmung (1102) zu formen, die an dem anderen des oberen konkaven Elements (102a) und des unteren Ringelements (102b) geformt ist.

5. Spritze nach Anspruch 4, **dadurch gekennzeichnet, dass** die ein oder mehreren Fixierelemente (802) entlang eines Umfangs von einem des oberen konkaven Elements (102a) und des unteren Ringelements (102b) radial voneinander beabstandet sind.

6. Spritze nach Anspruch 1, des Weiteren umfassend eine elastische Kappe (202), die über zumindest einen Teil eines Außenteils des Stempelkörpers (102) gestülpt ist.

7. Spritze nach Anspruch 1, des Weiteren umfassend eine Schulter (220), die an der Ausnehmung (104) geformt ist und ausgebildet ist, mit einer lasttragenden Oberfläche (220) des Kolbens zu koppeln.

## Revendications

1. Seringue comprenant:
un cylindre de seringue (201) définissant une chambre principale (200), et
un plongeur ayant un corps de plongeur (102) disposé à coulissement à l'intérieur de la chambre principale (200) du cylindre de seringue (201),
**caractérisée par le fait qu'**un évidement (104) est formé sur une paroi intérieure (122) du corps de plongeur (102), ledit évidement (104) étant configuré pour recevoir une partie de tête d'un piston, ledit évidement (104) comprenant une ou plusieurs brides (120) sur la paroi intérieure (122) qui font saillie radialement vers l'intérieur, le corps de plongeur (102) présentant un élément concave supérieur (102a) qui définit une première partie de l'évidement (104) et une première partie de ladite une ou des plusieurs brides (120) faisant saillie radialement vers l'intérieur, et un élément annulaire inférieur (102b) qui définit une deuxième partie de l'évidement (104) et une deuxième partie de ladite une ou des plusieurs brides (120) faisant saillie radialement vers l'intérieur, dans laquelle chacune de ladite une ou des plusieurs brides (120) faisant saillie radialement vers l'intérieur définit une arête se terminant sur un point culminant (121) pour définir un trajet de guidage en forme de dents de scie destiné à guider un mouvement rotatif de la partie de tête du piston dans l'évidement (104), et **par le fait que** ledit élément concave supérieur (102a) et ledit élément annulaire inférieur (102b) forment l'un avec l'autre un accouplement à engagement.

2. Seringue selon la revendication 1, **caractérisée par le fait que** le trajet de guidage en forme de dents de scie est configuré pour guider une ou plusieurs broches sur la partie de tête du piston.

3. Seringue selon la revendication 1, **caractérisée par le fait que** ladite une ou lesdites plusieurs brides (120) faisant saillie vers l'intérieur présentent le point culminant (121) configuré pour guider une ou plusieurs broches sur la partie de tête du piston dans le trajet de guidage.

4. Seringue selon la revendication 1, **caractérisée par le fait que** l'un des éléments concave supérieur (102a) et annulaire inférieur (102b) présente un ou plusieurs éléments de positionnement (802) configurés pour former l'accouplement à engagement avec un évidement de positionnement (1102) correspondant formé sur l'autre des éléments concave supérieur (102a) et annulaire inférieur (102b).

5. Seringue selon la revendication 4, **caractérisée par le fait que** ledit un ou lesdits plusieurs éléments de positionnement (802) sont espacés radialement les uns des autres le long d'une circonférence de l'un des éléments concave supérieur (102a) et annulaire inférieur (102b).

6. Seringue selon la revendication 1, comprenant en outre un capuchon élastique (202) qui est monté sur au moins une partie d'une partie extérieure du corps de plongeur (102).

7. Seringue selon la revendication 1, comprenant en outre un épaulement (220) qui est formé sur l'évidement (104) et configuré pour venir en prise avec une surface de support de charge (220) du piston.
